# EUROPÄISCHE PATENTSCHRIFT

(11) **EP 1 499 879 B1**
(45) Veröffentlichungstag und Bekanntmachung des Hinweises auf die Patenterteilung: **28.06.2006**
(21) Anmeldenummer: 03717025.5
(22) Anmeldetag: 23.04.2003
(51) Int. Cl.: G01N 25/14

(54) **VERFAHREN ZUR AUFNAHME DER SIEDEKURVE VON FLÜSSIGKEITEN SOWIE VORRICHTUNG ZUR DURCHFüHRUNG DIESES VERFAHRENS**
METHOD FOR RECORDING THE BOILING-POINT CURVE OF LIQUIDS AND DEVICE FOR CARRYING OUT SAID METHOD
PROCEDE POUR RELEVER LA COURBE DES POINTS D'EBULLITION DE LIQUIDES ET DISPOSITIF PERMETTANT DE METTRE EN OEUVRE CE PROCEDE

(30) Priorität: 24.04.2002 AT 2702002 U
(43) Veröffentlichungstag der Anmeldung: 26.01.2005
(60) Teilanmeldung: 05021996.3
(73) Patentinhaber: Grabner Instruments Messtechnik NFG. Gesellschaft m.b.H. & CO KG, 1220 Wien (AT)
(72) Erfinder: BURIAN, Matthias, A-2231 Strasshof a.d. Nordbahn (AT); ASCHAUER, Roland, A-1220 Wien (AT)
(74) Vertreter: Haffner, Thomas M.
(86) Internationale Anmeldenummer: PCT/AT2003/000118
(87) Internationale Veröffentlichungsnummer: WO 2003/091667

(56) Entgegenhaltungen:
- DE-A- 3 039 487
- FR-A- 2 815 413
- US-A- 3 399 116
- US-A- 4 250 739
- US-A- 4 528 635
- PATENT ABSTRACTS OF JAPAN vol. 012, no. 175 (P-707), 25. Mai 1988 (1988-05-25) -& JP 62 285052 A (IDEMITSU KOSAN CO LTD), 10. Dezember 1987 (1987-12-10) & DATABASE WPI Section Ch, Week 198804 Derwent Publications Ltd., London, GB; Class H04, AN 1988-024755 & JP 62 285052 A (IDEMITSU KOSAN CO LTD), 10. Dezember 1987 (1987-12-10)

## Beschreibung

Die Erfindung bezieht sich auf ein Verfahren zur Aufnahme der Siedekurve von Flüssigkeiten, insbesondere Erdölprodukten und/oder Lösungsmitteln, bei welchem eine Probemenge der zu analysierenden Flüssigkeit verdampft und anschließend kondensiert wird, wobei die Dampftemperatur und die jeweils verdampfte Menge der Flüssigkeit überwacht wird und die Probemenge dosiert wird, sowie eine Vorrichtung zur Durchführung dieses Verfahrens.

Die Destillationseigenschaften von Flüssigkeiten und insbesondere von Erdölprodukten, wie beispielsweise von Treibstoff, sind in zahlreichen Produktspezifikationen festgelegt. Im Fall von Treibstoffen soll auf diese Weise sichergestellt werden, dass Motoren in den üblicherweise vorkommenden Leistungs- und Temperaturbereichen mit einem entsprechenden Treibstoff arbeiten. Weltweit anerkannt gibt es in diesem Zusammenhang ein Standardverfahren der Siedeanalyse für Erdölprodukte, das von ASTM und ISO anerkannt ist. Bei diesem Verfahren werden 100 ml der zu untersuchenden Flüssigkeit in einem beheizten Destillierkolben aus Glas destilliert und in einem gekühlten Kondenserrohr kondensiert. Das Kondensat wird in einem graduierten Glaszylinder aufgefangen. Zwischen Siedebeginn und Siedeende werden Dampftemperatur und bisher kondensiertes Volumen bestimmt (Siedekurve). Das Volumen des Destillationsrückstandes, der nach Abschalten der Heizung im Destillierkolben verbleibt, wird mit einem Messzylinder bestimmt, und die Siedekurve wird entsprechend korrigiert.

Diese Methode wurde um 1920 entwickelt. Die Regelung der Heizung, das Ablesen der Dampftemperatur und die Bestimmung des korrespondierenden Kondensatvolumens wurde dabei vom Bediener der Apparatur durchgeführt.

Die DE3039487 offenbart ein kontinuierlich ablaufendes Kurzweg-Destillationsverfahren zur Aufnahme der Siedekurve und eine entsprechende Vorrichtung für hochsiedende Flüssigkeiten, insbesondere Erdölprodukte. Aus einem Vorratsgefäss wird die Probemenge über eine als Zahnradpumpe ausgebildete Dosierpumpe einer beheizten Fläche zugeführt und auf dieser als dünner, ständig bewegter Film ausgebreitet. Der Dampf schlägt sich an einem im kurzen Abstand davon angeordneten Kondensator nieder. Das Destillat steht am Destillatauslauf, die unveränderten Rückstandsanteile am Rückstandsauslauf zur Verfügung. Das Vorratsgefäss, die Dosierpumpe, der Zuführung der Probenmenge dienenden Bauteile, die Kurzwegdestillationsanlage, der Destillatauslauf und der Rückstandsauslauf werden unabhängig voneinander beheizt und wahlweise auf unterschiedliche Temperaturen eingestellt. Destillats- und Rückstandsfraktionen werden gewogen.

In der US 4,528,635 wird eine automatische Destillationsapparatur beschrieben. Diese Apparatur verwendet die gleichen Destillierkolben, Kondenser und Glaszylinder wie die ursprüngliche manuelle Methode. In der Apparatur wurde eine Vorrichtung zur Bestimmung des kondensierten Volumens eingebaut, sowie ein Microprozessor, der die Heizung und Kühlung kontrolliert sowie automatisch Messdaten aufnimmt. Grundsätzlich jedoch blieb die Methode unverändert.

Diese Standardmethode hat eine Reihe von Nachteilen für die Anwender. Das Probenvolumen ist mit 100 ml hoch, Probe und Destillationsrückstand müssen händisch abgemessen und eingefüllt werden, die Glaskolben und -zylinder sind zerbrechlich und müssen nach jeder Destillation gereinigt werden, die Messzeit ist hoch, und die Apparatur ist großvolumig und kann nur im Labor verwendet werden.

Die vorliegende Erfindung zielt nun darauf ab, ein Verfahren und eine Vorrichtung zu schaffen, bei welcher mit wesentlich geringeren Baumaßen die Möglichkeit geboten wird, die Einrichtung als portable Einrichtung auszubilden, wobei gleichzeitig das Verfahren so geführt werden soll, dass das Ausmaß einer manuellen Einflussnahme auf ein Minimum beschränkt wird. Neben der erfindungsgemäß angestrebten Miniaturisierung und Automatisierung zielt die Erfindung gleichzeitig darauf ab, bei einer Verkürzung der Messzeit und gleichzeitiger Verringerung der erforderlichen Probemenge Resultate zu liefern, welche der ASTM-Standardmethode äquivalent sind und auf Grund der weitestgehenden Automatisierung ein höheres Maß an Genauigkeit und Reproduzierbarkeit der Messergebnisse liefern. Insbesondere zielt die Erfindung darauf ab eine vollautomatische Führung des Verfahrens zu ermöglichen und die gesamte für die Durchführung des Verfahrens erforderliche Einrichtung in ihren Baumaßen so weit zu reduzieren, dass sie in einem konventionellen Kraftfahrzeug, beispielsweise im Kofferraum, untergebracht werden und vom Bordnetz eines derartigen Kraftfahrzeuges mit Energie versorgt werden kann.

Zur Lösung dieser Aufgabe besteht das erfindungsgemäße Verfahren im Wesentlichen darin, dass die Probemenge von einer Dosiereinrichtung in eine Probenschale dosiert wird, welche mittels eines Hubantriebes in eine mit der Destilliereinrichtung gasdichte Verbindung bewegt und angepresst wird, dass die Dosiereinrichtung und der Aufnahmeraum für das Kondensat auf ein definiertes und vorzugsweise gleiches Temperaturniveau gebracht wird und dass das kondensierte Volumen in an sich bekannter Weise volumetrisch und der Destillationsrückstand der Probemenge durch Wägen bestimmt wird. Dadurch, dass die Probemenge von einer Pumpe dosiert wird, wird eine erste üblicherweise manuelle Einflussname vermieden und ein hohes Maß an Präzision bei der Beschickung der zur Durchführung des Verfahrens geeigneten Vorrichtung erzielt. Gleichzeitig mit der Automatisierung dieser Befüllung beziehungsweise Dosierung der Probemenge wird nun dadurch, dass die Fülleinrichtung und der Aufnahmeraum für das Kondensats auf ein definiertes und vorzugsweise gleiches Temperaturniveau gebracht wird, eine Fehlerquelle eliminiert und eine exakte Volumsbestimmung des Kondensats beziehungsweise eine exakte Bestimmung des aus der Probemenge bereits destillierten Produktes ermöglicht. Dadurch, dass nun der Destillationsrückstand der Probemenge durch Wägen bestimmt wird, lässt sich mit hoher Präzision auf entsprechende Volumseinheiten umrechnen und gemeinsam mit der volumetrischen Bestimmung des kondensierten Volumens bei kleinen Probemengen ein hochpräzises Messergebnis gewährleisten, welches frei von durch Manipulation oder Temperaturschwankungen hervorgerufenen Verzerrungen in hoher Präzision zur Verfügung steht. Dadurch, dass die Probemenge in eine Probenschale gepumpt wird, welche mittels eines Hubantriebes in eine mit der Destilliereinrichtung gasdichte Verbindung bewegt und angepresst wird, wird eine vollständige Automatisierung erreicht. Eine derartige Probenschale kann, wie es einer bevorzugten Ausbildung entspricht, in einfacher Weise aus Metall bestehen und daher als billiger Wegwerfartikel ausgeführt werden. Es entfallen somit die durch unzureichende Reinigung von Glasapparaturen hervorgerufenen Verzerrungen und Fehler, da es genügt, eine derartig billige und kleine Probenschale nach dem Siedeende einfach zu verwerfen und eine neue Schale für die nächste Bestimmung zu verwenden. Die einfache mechanische und voll automatische Anpressung der Probenschale an die Destilliereinrichtung kann umgekehrt auch dazu verwendet werden, um die Probenschale nach Ende der Destillation wiederum abzusenken und einer Wägevorrichtung zuzuführen, um den Destillationsrückstand mit hoher Genauigkeit zu bestimmen.

Eine besonders einfache Reinigung und eine weitere Automatisierung des erfindungsgemäßen Verfahrens läßt sich dadurch erzielen, dass die Fülleinrichtung für das Dosieren der Probemenge nach dem Ende des Messvorganges über wenigstens ein Ventil zum Absaugen des Kondensates geschaltet wird, wobei, wie bereits erwähnt, eine Reinigung der Probenschale entfallen kann, wenn, wie erfindungsgemäß vorgeschlagen, die Probenschale nach ihrer einmaligen Verwendung verworfen wird.

Die thermostatierte Fülleinrichtung erlaubt somit nicht nur ein präzises Dosieren der Probenmenge sondern gleichzeitig auch ein entsprechendes Abziehen des Destillats und erforderlichenfalls eine entsprechende Spülung bzw. Reinigung. Insbesondere bei der bevorzugt eingesetzten Vorrichtung zur Bestimmung des Volumens des Kondensats kann diese Pumpe auch in besonders einfacher Weise zur Einstellung des Meniskus dienen, dessen Höhe in der Folge überwacht und für die volumetrische Bestimmung des Kondensats ausgewertet werden kann.

Insgesamt wird im Rahmen des erfindungsgemäßen Verfahrens bevorzugt so vorgegangen, dass Probe in einer Menge von 1 bis 12 ml, vorzugsweise etwa 6 ml, eingesetzt wird, dass die Destillationsrate so gewählt wird, dass die Zeit zwischen Siedebeginn und Siedeende weniger als 15 Minuten beträgt und dass der durch Wägen ermittelte Destillationsrückstand auf Volumen umgerechnet wird, wobei Messzeiten auf etwa die Hälfte der bisher bekannten Standardmesszeiten reduziert werden können und mit weniger als einem Zehntel des bisher verwendeten Volumens der Probenflüssigkeit das Auslangen gefunden werden kann. Wesentlich ist in diesem Zusammenhang die entsprechende Wahl der Parameter in den genannten Grenzen, da im Rahmen dieser Parameter die Möglichkeit geboten wird, unmittelbar vergleichbare Ergebnisse zu erzielen, welche mit den bekannten Standardmesskurven für die Siedeanalyse, soweit sie standardisiert vorliegen, unmittelbar vergleichbar sind.

Die erfindungsgemäße Vorrichtung zur Durchführung dieses Verfahrens ist im Wesentlichen dadurch gekennzeichnet, dass eine Pumpe zum Einfüllen einer Probemenge mit einer Leitung zu einer Probenschale verbunden ist, dass die Probenschale auf einer verfahrbaren Unterlage angeordnet ist, welche mit einem als Hubantrieb ausgebildeten Stellantrieb verbunden ist, durch welchen der Rand der Probenschale an den Rand der Anschlussöffnung einer an die Probenschale anschließenden Destillierkolonne gedrückt werden kann und dass die Pumpe und der Aufnahmeraum für das Kondensat bzw. der Kondenser aus gut wärmeleitendem Material, insbesondere Metall, bestehen, und mit einer Temperaturregeleinrichtung verbunden sind. Dadurch, dass die Fülleinrichtung ebenso wie die Destillationseinrichtung und insbesondere derjenige Teil der Destillationseinrichtung, in welchem die volumetrische Bestimmung des Kondensats vorgenommen wird, nämlich der Kondenser, aus gut wärmeleitendem Material besteht, ist es möglich, auch bei besonders kleinbauenden Einrichtungen eine hinreichend homogene Temperaturverteilung sicherzustellen und eine entsprechende Temperaturregeleinrichtung mit hoher Präzision zu verwenden. Es gelingt somit auch bei kleinbauenden Einrichtungen durch entsprechende Thermostatierung, die entsprechenden Fehler gering zu halten und die Präzision der Ergebnisse zu steigern, sodass insgesamt eine portable Vorrichtung realisierbar wird. Dadurch, dass nun die Probenschale auf einer verfahrbaren Unterlage angeordnet ist, welche mit einem Stellantrieb verbunden ist, wird die Möglichkeit geschaffen, eine kostengünstige Alternative für Glasgefäße zu verwenden, und insbesondere die Möglichkeit geschaffen, eine derartige Probenschale nach ihrer einmaligen Verwendung auch zu verwerfen, da sie einen hinreichend billigen Bauteil darstellt. Die Probenschale kann hierbei mittels des Stellantriebes gegen die Destillationseinrichtung angestellt werden und durch Anpressen mit der Destillationseinrichtung entsprechend gasdicht verbunden werden, wobei gleichzeitig die Probenschale entsprechend beheizt werden kann, um eine Destillation zu ermöglich. Weiters erlaubt es der verfahrbare Stellantrieb, die Probenschale am Siedeende wieder abzuziehen und einer Wägeeinrichtung zuzuführen, um die entsprechende Bestimmung des Destillationsrückstandes mit hoher Präzision zu ermöglichen.

Im Sinne einer kleinbauenden portablen Einrichtung, welche auch mit einfachem Batteriebetrieb mit Energie versorgt werden kann, ist die Ausbildung mit Vorteil so getroffen, dass die Temperaturregeleinrichtung als elektrische Kühl- und/oder Heizeinrichtung, insbesondere unter Verwendung von Peltierelementen, ausgebildet ist. Derartige Peltierelemente werden in der Regel so gepolt, dass sie als Kühleinrichtung zum Einsatz gelangen. Durch Umpolung kann aber bei entsprechend ungünstigen Witterungsbedingungen auch eine entsprechend höhere Temperatur gewählt werden, um zu mit Standardtests vergleichbaren Ergebnissen zu gelangen.

Eine vollständig autonome und tragbare Vorrichtung ergibt sich dann, wenn die Fülleinrichtung und die Destillationseinrichtung in einem gemeinsamen, tragbaren Gehäuse angeordnet sind.

Die entsprechende Kompensation beziehungsweise Korrektur der volumetrischen Messergebnisse kann in einfacher Weise dadurch gewährleistet werden, dass die Fülleinrichtung und der Kondenser Temperatursensoren aufweisen.

Der Stellantrieb für die Probenschale kann in einfacher Weise als Getriebemotor ausgebildet sein, wodurch auch ein entsprechender Anpressdruck beim Anheben der Probenschale gewährleistet werden kann. Zur Erzielung eines dichten Abschlusses zwischen Probenschale und Destillierkolonne ist die Ausbildung mit Vorteil so getroffen, dass der Rand der Probenschale und der Rand der Anschlussöffnung der Destillierkolonne konisch, hohlkonisch oder ballig zur gasdichten Verbindung der Probenschale mit der Destilliereinrichtung unter Anwendung des vom Stellantrieb erzeugten Anpressdruckes ausgebildet sind.

Um auch hier thermische Fehler, welche zu einer Verzerrungen der Messwerte führen könnten, mit Sicherheit zu vermeiden, ist die Ausbildung mit Vorteil so getroffen, dass die Destillierkolonne von einer Isolation umgeben ist.

Eine besonders vorteilhafte Ausgestaltung der volumetrischen Messeinrichtung in einer im wesentlichen aus Metall bestehenden Destillationseinrichtung lässt sich dadurch erzielen, dass, wie es einer bevorzugten Ausbildung entspricht, der Kondenser einen axialen Bereich mit verringertem Durchmesser aus einem für Licht, insbesondere Infrarotlicht durchsichtigem Material, insbesondere Glas aufweist, an welchen ein einen axial beweglichen Kolben aufnehmender Bereich mit größerer lichter Weite anschließt. Ein derartiger beispielsweise aus Glasröhrchen ausgebildeter Abschnitt kann entsprechend geringen lichten Querschnitt aufweisen, sodass bereits geringe Volumsänderungen eine entsprechende Veränderung des Flüssigkeitsspiegels, beziehungsweise des Meniskus, zu Folge haben. Um dennoch eine entsprechend größere Menge des Kondensats aufnehmen zu können, schließt an ein derartiges Glasröhrchen der axial bewegliche Kolben in einem Zylinder mit entsprechend größerer lichter Weite an, wobei die Messung in einfacher Weise als Messung des Kolbenstellweges erfolgen kann, über welchen auf das entsprechende Volumen geschlossen werden kann. Zu diesem Zweck ist mit Vorteil die Ausbildung so getroffen, dass der axial bewegliche Kolben mit einem Stellantrieb, insbesondere einem Schrittmotor oder einem Getriebemotor mit einem Drehstellungsgeber verbunden ist, welcher in Abhängigkeit von den Signalen eines optischen Detektors antreibbar ist, wobei vorzugsweise der optische Signalgeber im Bereich des axialen Bereiches aus für Licht durchsichtigem Material angeordnet ist und zur Detektion des Meniskus der kondensierten Flüssigkeit ausgebildet ist und dass der Stellantrieb des Kolbens zur Korrektur der Position des Meniskus antreibbar ist.
Wie bereits erwähnt, kann in besonders einfacher Weise die Destilliereinrichtung aus Edelstahl, Messing oder Titan und die Probenschale aus Metall, vorzugsweise Aluminium oder Kupfer bestehen. Eine derartige Ausbildung zeichnet sich durch entsprechend kostengünstige Gestaltung der Probenschale bei gleichzeitig einfacher homogener Temperaturverteilung aus.

Die ASTM-Standardmethode ist auf eine Siedeanalyse unter atmosphärischem Druck ausgelegt. Mit Vorteil ist daher die Ausbildung so getroffen, dass ein Drucksensor, insbesondere ein piezoresistiver Drucksensor zur Bestimmung des Luftdruckes vorgesehen ist und dass die Destillationseinrichtung im Bereich des Kondensers in offener Verbindung zur Atmosphäre ausgebildet ist, sodass eine entsprechend kompensierende Rechnung auf der Basis der Druckmesswerte jederzeit gelingt. Eine vollständige Erfassung aller Messwerte im gleichen Gerät und insbesondere auch eine gewichtsmäßige Erfassung des in der Probenschale enthaltenen Destillationsrückstandes gelingt mit Vorteil dann, wenn die Ausbildung so getroffen ist, dass im Gehäuse eine Waage angeordnet ist.

Die vollautomatische Durchführung des im Rahmen einer derartigen Vorrichtung durchführbaren Verfahrens gelingt in besonders einfacher weise dann, wenn die Messwerte über Leitungen einem Mikroprozessor zugeführt sind und dass eine Anzeige bzw. Ausgabeeinrichtung für die Messergebnisse vorgesehen ist.

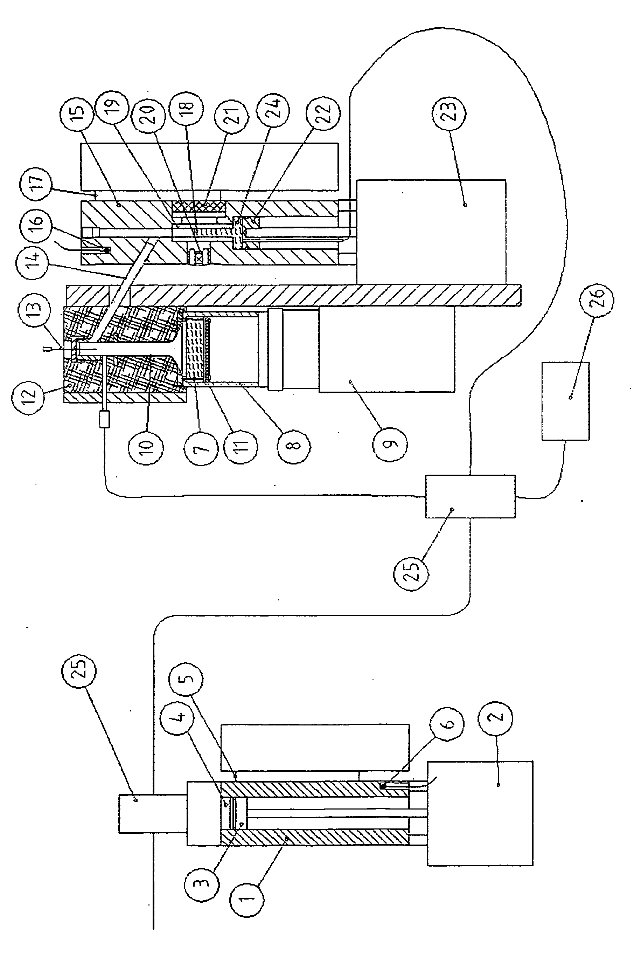

Die Erfindung wird nachfolgend anhand eines in der Zeichnung schematisch dargestellten Ausführungsbeispieles der erfindungsgemäßen Vorrichtung sowie einer verfahrensbeschreibung näher erläutert.

Die Fülleinrichtung 1 besteht aus einem mit einem Schrittmotor oder Getriebemotor mit Drehgeber 2 angetriebenen Kolben 3, der in einer vernickelten Aluminiumkammer 4 bewegt wird und mit einem O-Ring gedichtet wird. Volumsauflösung ist besser als 1_. Die Temperatur der Fülleinrichtung 1 wird mit einem Peltierelement 5 reguliert und mit einem Temperatursensor 6, vorzugsweise einem Pt-100-Widerstandssensor, gemessen.

Die Probenschale 7 besteht aus tiefgezogenem Aluminium. Dadurch ist sie billig und wird nach der Messung weggeworfen, sodass eine aufwendige Reinigung entfällt. Die Probenschale 7 wird über einen Träger 8, der mit einem Getriebemotor 9 bewegt werden kann, gegen die Edelstahl-Destillationssäule 10 gedrückt. Im Träger 8 ist auch die elektrische Heizung 11 integriert. Auf die Destillationssäule 10 ist ein abgeschrägter Ring gedreht, an den die Probenschale gedrückt wird. Dadurch entsteht eine luftdichte Messkammer. Die Destillationssäule 10 ist außen mit Mineralwolle 12 isoliert, sodass Unterschiede der Umgebungstemperatur keinen Einfluss auf die Dampftemperatur haben können.

Die Dampftemperatur wird mit dem Thermoelement 13 bestimmt. Seitlich an der Destillationssäule 10 befindet sich ein Auslass 14, der in den Kondenser 15 mündet. Der Kondenser 15 besteht aus Aluminium und ist oben offen, sodass die Destillation unter dem vorherrschenden Luftdruck stattfindet. Die Kondensertemperatur wird mit einem Temperatursensor 16, vorzugsweise einem Pt-100-Widerstandsthermometer, gemessen und mit Hilfe eines Peltierele-mentes 17 konstant gehalten.

Im Kondenser eingebaut ist ein System 18 zur Volumsdetektion. Dieses System besteht aus einem Glasrohr 19, das in den Kondenser eingebaut ist und dessen Temperatur daher gleich der des Kondensers 15 ist. Zwei LEDs 20 beleuchten das Glasrohr 19 mit einem etwa parallelen Strahl. Ein optischer Zeilendetektor 21 (vorzugsweise ein Photodiodenarray) ist so angebracht, dass die Dioden im Brennpunkt der Zylinderlinse sitzen, die das Glasrohr 19 bildet, wenn es mit Probe gefüllt ist. Ist das Glasrohr 19 nun bis etwa zur Hälfte mit Flüssigkeit gefüllt, wird der Lichtstrahl der LEDs 20 in dem Bereich, in dem sich die Flüssigkeit befindet, auf den Zeilendetektor 21 fokussiert. Oberhalb der Grenzfläche Flüssigkeit-Luft (Meniskus) ist diese Zylinderlinsenwirkung nicht gegeben. Dadurch registriert der Detektor 21 unterhalb des Meniskus erhöhte Lichtintensität, während oberhalb des Meniskus die Intensität niedriger ist.

Steigt der Meniskus im Glasrohr 19, so verändert sich ebenso die Intensitätsverteilung am Zeilendetektor 21. Die Ortsauflösung für die Bestimmung der Meniskusposition ist besser als 0,6 mm.

Die Position des Meniskus kann durch den Kolben 22, der von einem Schrittmotor oder einem Getriebemotor mit Drehgeber 23 in einer zylindrischen Kammer 24 bewegt wird, verändert werden. Die Änderung der Höhe des Meniskus nach einer Bewegung des Kolbens 22 wird mit dem Zeilendetektor 21 bestimmt.

Wenn nun Dampf im Kondenser kondensiert, steigt der Meniskus im Glasrohr 19 an. Wenn dieser Anstieg einen gewissen Wert erreicht, wird der Kolben 22 um einen vorbestimmten Betrag nach unten bewegt, wodurch der Meniskus wieder sinkt. Auf diese Weise wird der Meniskus immer im Bereich des Zeilendetektors 21 gehalten.

Aus dem bekannten Innendurchmesser des Glasrohres 19 und der Veränderung der Meniskushöhe wird das Volumen der kondensierten Probe bestimmt. Die Volumsauflösung ist dabei besser als 10µ_ und beträgt mindestens 0,1% des Füllvolumens.

Zur Verteilung der Probe in der Apparatur dienen Ventile 25. Ein Abfallbehälter 26 nimmt verbrauchte Probe auf.

Alle Sensoren, Motoren, Peltierelemente, Ventile und die Heizung werden von einem Mikroprozessor gesteuert und überwacht, sodass der Messvorgang nach einem vorgegebenen Programm vollautomatisch ohne Eingreifen des Bedieners abläuft.

Die ganze Vorrichtung kann in ein tragbares Gehäuse eingebaut werden.

### Messvorgang:

Zu Beginn einer Messung wird eine frische Probeschale 7 auf den Träger 8 gelegt und dadurch mit Hilfe des Motors 9 gegen die Destillationssäule 10 gepresst. Die Fülleinrichtung 1 und der Kondenser 15 werden auf gleiche Temperatur geregelt. Dann wird Probe mit der Fülleinrichtung 1 angesaugt. Ein vorgegebenes Volumen, vorzugsweise 6 ml, wird in die Probenschale 7 dosiert. Ebenso wird ein kleines Volumen in das Volumsdetektionssystem 18 gefüllt, sodass sich der Meniskus im Glasrohr 19 im Detektionsbereich des Zeilendetektors 21 befindet.

Die Heizung 11 wird nun aufgedreht. Die Probe beginnt kurz darauf zu sieden und zu verdampfen. Die Temperatur des aufsteigenden Dampfes wird mit dem Thermoelement 13 kontinuierlich gemessen. Die Leistung der Heizung hängt von dieser Dampftemperatur ab und wird vom Mikroprozessor so geregelt, dass sie mit steigender Dampftemperatur zunimmt.

Der Dampf tritt durch den Auslass 14 in den Kondenser 15 über und kondensiert dort wieder. Das kondensierte Volumen wird laufend mit dem Volumsdetektionssystem 18 gemessen.

Wenn die Probe vollständig verdampft ist, wird kein Dampf mehr nachgeliefert, und die Temperatur des Thermoelements 13 sinkt. Dann wird die Heizung 11 abgedreht. Sobald die Temperatur unter einen Schwellwert sinkt, fährt der Träger 8 mit der Probenschale 7 nach unten, und die Probenschale kann herausgenommen, gewogen und danach weggeworfen werden. Die verbrauchte Probe im Kondenser 15 wird über das Füllsystem 1 abgesaugt und in den Abfallbehälter 26 gefüllt.

Das Gewicht der Probenschale mit Rückstand wird eingegeben. Daraus wird vom Mikroprozessor der Destillationsrückstand berechnet, und die Siedekurve wird entsprechend korrigiert. Die Messresultate können auf einem eingebauten Display angezeigt, ausgedruckt oder auf einen Computer überspielt werden.

Die gesamte Messzeit beträgt etwa die Hälfte der für das ASTM-Standardverfahren notwendigen Zeit.

## Patentansprüche

1. Verfahren zur Aufnahme der Siedekurve von Flüssigkeiten, insbesondere Erdölprodukten und/oder Lösungsmitteln, bei welchem eine Probemenge der zu analysierenden Flüssigkeit verdampft und anschließend kondensiert wird, wobei die Dampftemperatur und die jeweils verdampfte Menge der Flüssigkeit überwacht wird, wobei die Probemenge von einer Dosiereinrichtung dosiert wird, **dadurch gekennzeichnet, dass** die Probemenge in eine Probenschale dosiert wird, welche mittels eines Hubantriebes in eine mit der Destilliereinrichtung gasdichte Verbindung bewegt und angepresst wird, dass die Dosiereinrichtung und der Aufnahmeraum für das Kondensat auf ein definiertes und vorzugsweise gleiches Temperaturniveau gebracht wird und dass das kondensierte Volumen in an sich bekannter Weise volumetrisch und der Destillationsrückstand der Probemenge durch Wägen bestimmt wird.

2. Verfahren nach Anspruch 1, **dadurch gekennzeichnet, dass** die Dosiereinrichtung für das Dosieren der Probemenge nach dem Ende des Messvorganges über wenigstens ein Ventil zum Absaugen des Kondensates geschaltet wird.

3. Verfahren nach Anspruch 1 oder 2, **dadurch gekennzeichnet, dass** die Probenschale nach ihrer einmaligen Verwendung verworfen wird.

4. Verfahren nach Anspruch 1, 2 oder 3, **dadurch gekennzeichnet, dass** Probe in einer Menge von 1 bis 12 ml, vorzugsweise etwa 6 ml, eingesetzt wird, dass die Destillationsrate so gewählt wird, dass die Zeit zwischen Siedebeginn und Siedeende weniger als 15 Minuten beträgt und dass der durch Wägen ermittelte Destillationsrückstand auf Volumen umgerechnet wird.

5. Vorrichtung zur Durchführung des Verfahrens nach einem der Ansprüche 1 bis 4, **dadurch gekennzeichnet, dass** eine Pumpe (1) zum Einfüllen einer Probemenge mit einer Leitung zu einer Probenschale (7) verbunden ist, dass die Probenschale (7) auf einer verfahrbaren Unterlage (8) angeordnet ist, welche mit einem als Hubantrieb ausgebildeten Stellantrieb (9) verbunden ist, durch welchen der Rand der Probenschale (7) an den Rand der Anschlussöffnung einer an die Probenschale (7) anschließenden Destillierkolonne (10) gedrückt werden kann und dass die Pumpe (1) und der Aufnahmeraum für das Kondensat bzw. der Kondenser (15) aus gut wärmeleitendem Material, insbesondere Metall, bestehen, und mit einer Temperaturregeleinrichtung (5,6,16,17) verbunden sind.

6. Vorrichtung nach Anspruch 5, **dadurch gekennzeichnet, dass** die Temperaturregeleinrichtung als elektrische Kühl- und/oder Heizeinrichtung, insbesondere unter Verwendung von Peltierelementen (5,17), ausgebildet ist und ebenso wie die Pumpe (1) und die Destillationseinrichtung in einem gemeinsamen, tragbaren Gehäuse angeordnet ist.

7. Vorrichtung nach einem der Ansprüche 5 oder 6, **dadurch gekennzeichnet, dass** die Pumpe (1) und der Kondenser (15) Temperatursensoren (6,16) aufweisen.

8. Vorrichtung nach einem der Ansprüche 5, 6 oder 7, **dadurch gekennzeichnet, dass** der Stellantrieb (9) für die Probenschale (7) als Getriebemotor ausgebildet ist.

9. Vorrichtung nach einem der Ansprüche 5 bis 8, **dadurch gekennzeichnet, dass** der Rand der Probenschale (7) und der Rand der Anschlussöffnung der Destillierkolonne (10) konisch, hohlkonisch oder ballig zur gasdichten Verbindung der Probenschale (7) mit der Destilliereinrichtung unter Anwendung des vom Stellantrieb (9) erzeugten Anpressdruckes ausgebildet sind.

10. Vorrichtung nach einem der Ansprüche 5 bis 9, **dadurch gekennzeichnet, dass** die Destillierkolonne (10) von einer Isolation (12) umgeben ist.

11. Vorrichtung nach einem der Ansprüche 5 bis 10, **dadurch gekennzeichnet, dass** der Kondenser (15) einen axialen Bereich mit verringertem Durchmesser aus einem für Licht, insbesondere Infrarotlicht durchsichtigem Material, insbesondere Glas aufweist, an welchen ein einen axial beweglichen Kolben (22) aufnehmender Bereich mit größerer lichter Weite anschließt.

12. Vorrichtung nach Ansprüche 11, **dadurch gekennzeichnet, dass** der axial bewegliche Kolben (22) mit einem Stellantrieb, insbesondere einem Schrittmotor oder einem Getriebemotor mit einem Drehstellungsgeber (23) verbunden ist, welcher in Abhängigkeit von den Signalen eines optischen Detektors (21) antreibbar ist.

13. Vorrichtung nach einem der Ansprüche 5 bis 12, **dadurch gekennzeichnet, dass** ein optischer Signalgeber (20) im Bereich des axialen Bereiches aus für Licht durchsichtigem Material angeordnet ist und zur Detektion des Meniskus der kondensierten Flüssigkeit ausgebildet ist und dass der Stellantrieb des Kolbens (22) zur Korrektur der Position des Meniskus antreibbar ist.

14. Vorrichtung nach einem der Ansprüche 5 bis 13, **dadurch gekennzeichnet, dass** die Destilliereinrichtung aus Edelstahl, Messing oder Titan und die Probenschale (7) aus Metall, vorzugsweise Aluminium oder Kupfer besteht.

15. Vorrichtung nach einem der Ansprüche 5 bis 14, **dadurch gekennzeichnet, dass** ein Drucksensor, insbesondere ein piezoresistiver Drucksensor zur Bestimmung des Luftdruckes vorgesehen ist und dass die Destillationseinrichtung im Bereich des Kondensers (15) in offener Verbindung zur Atmosphäre ausgebildet ist.

16. Vorrichtung nach einem der Ansprüche 5 bis 15, **dadurch gekennzeichnet, dass** im Gehäuse eine Waage angeordnet ist, welche zum Abwiegen des Destillationsrückstandes der Probemenge ausgebildet ist.

17. Vorrichtung nach einem der Ansprüche 5 bis 16, **dadurch gekennzeichnet, dass** die Messwerte über Leitungen einem Mikroprozessor zugeführt sind, welcher zur Auswertung und Berechnung von Messergebnissen ausgebildet ist, und dass eine Anzeige bzw. Ausgabeeinrichtung für die Messergebnisse vorgesehen ist.

## Claims

1. A method for recording the boiling curve of liquids, in particular petroleum products and/or solvents, in which a sample amount of the liquid to be analyzed is evaporated and subsequently condensed, wherein the vapor temperature and the respectively evaporated amount of liquid are monitored, and wherein the sample amount is dosed by a metering unit, **characterized in that** the sample amount is pumped into a sample dish which is moved by the aid of a lifting drive into a gas-tight connection with the distillation apparatus and pressed at the same, that the metering unit and the condensate collection chamber are brought to a defined, and preferably identical, temperature level, and that the condensed volume is determined volumetrically in a manner known per se and the distillation residue of the sample amount is determined by weighing.

2. A method according to claim 1, **characterized in that** the metering unit for the dosing of the sample amount, after completion of the measuring procedure, via at least one valve is switched to remove the condensate by suction.

3. A method according to claim 1 or 2, **characterized in that** the sample dish is discarded after one-time use.

4. A method according to any one of claims 1,2 or 3, **characterized in that** sample is used in an amount of from 1 to 12 ml, preferably about 6 ml, that the distillation rate is chosen such that the time between the onset of boiling and the end of boiling is less than 15 minutes, and that the distillation residue determined by weighing is converted to volume.

5. A device for carrying out the method according to any one of claims 1 to 4, **characterized in that** a pump (1) for filling in a sample amount is connected with a duct leading to a sample dish (7), that the sample dish (7) is arranged on a movable support (8), which is connected with an adjustment drive (9) in form of a lifting drive, by which the edge of the sample dish (7) can be pressed at the edge of the connection opening of a distillation column (10) that follows the sample dish (7) and that the pump (1) and the condensate collection chamber or the condenser (15), are made of well heat-conducting material, particularly metal, and are connected to a temperature control equipment (5,6,16,17).

6. A device according to claim 5, **characterized in that** the temperature controller is designed as an electric cooler and/or heater using, in particular, Peltier elements (5, 17) and just as the pump (1) and the distillation apparatus is arranged in a common portable housing.

7. A device according to any one of claims 5 or 6, **characterized in that** temperature sensors (6, 16) are provided in both the pump (1) and the condenser (15).

8. A device according to any one of claims 5,6 or 7, **characterized in that** the adjustment drive (9) for the sample dish (7) is designed as a geared motor.

9. A device according to any one of claims 5 to 8, **characterized in that** the edge of the sample dish (7) and the edge of the connection opening of the distillation column (10) are designed to be conical, hollow-conical or ball-shaped, so as to ensure the gas-tight connection of the sample dish (7) with the distillation apparatus while applying the pressing pressure created by the adjustment drive (9).

10. A device according to any one of claims 5 to 9, **characterized in that** the distillation column (10) is surrounded by an insulation means (12).

11. A device according to any one of claims 5 to 10, **characterized in that** the condenser (15) comprises an axial zone with reduced diameter of a material transparent to light and, in particular, infrared light, particularly glass, which is followed by a zone having a larger clear width adapted to receive an axially movable piston (22).

12. A device according to claim 11, **characterized in that** the axially movable piston (22) is connected with an adjustment drive, particularly a stepper motor or geared motor including a rotary encoder (23), which is actuatable as a function of the signals transmitted by an optical detector (21).

13. A device according to any one of claims 5 to 12, **characterized in that** an optical signal transmitter (20) is arranged in the region of the axial zone of light-transparent material and designed for the detection of the meniscus of the condensed liquid, and that the adjustment drive of the piston (22) is actuatable for the correction of the position of the meniscus.

14. A device according to any one of claims 5 to 13, **characterized in that** the distillation apparatus is made of stainless steel, brass or titanium, and the sample dish (7) is made of metal, preferably aluminium or copper.

15. A device according to any one of claims 5 to 14, **characterized in that** a pressure sensor, particularly a piezoresistive pressure sensor, is provided for the determination of the air pressure, and that the distillation apparatus in the region of the condenser (15) is designed with an open connection to the atmosphere.

16. A device according to any one of claims 5 to 15, **characterized in that** a balance designed for the weighing of the distillation residue of the sample amount is arranged within the housing.

17. A device according to any one of claims 5 to 16, **characterized in that** the measuring values are fed via lines to a microprocessor designed for the evaluation and calculation of measuring results, and that a display or output means for the measuring results is provided.

## Revendications

1. Procédé pour enregistrer la courbe d'ébullition de liquides, en particulier de produits pétroliers et/ou de solvants, dans lequel une quantité d'échantillon du liquide à analyser est évaporée puis condensée, où la température de vapeur et la quantité de liquide évaporée à chaque fois sont surveillées, dans lequel la quantité d'échantillon est dosée par un dispositif de dosage, **caractérisé en ce que** la quantité d'échantillon est pompée dans un récipient d'échantillon qui, au moyen d'un organe de levage, est déplacé et appuyé dans une liaison étanche aux gaz avec le dispositif de distillation et **en ce que** le dispositif de remplissage et l'espace de réception pour le condensat sont amenés à un niveau de température défini et de préférence identique et **en ce que** le volume condensé est déterminé volumétriquement de manière connue en soi et le résidu de distillation de la quantité d'échantillon est déterminé de manière connue en soi par pesée.

2. Procédé selon la revendication 1 **caractérisé en ce que** le dispositif de dosage pour le dosage de la quantité d'échantillon est accouplé après la fin du processus de mesure par le biais d'au moins une vanne pour l'aspiration du condensat.

3. Procédé selon la revendication 1 ou 2 **caractérisé en ce que** le récipient d'échantillon est jeté après son utilisation unique.

4. Procédé selon la revendication 1,2 ou 3 **caractérisé en ce que** l'échantillon est utilisé en une quantité de 1 à 12 ml, de préférence d'environ 6 ml, **en ce que** la vitesse de distillation est choisie de telle manière que la durée entre le début de l'ébullition et la fin de l'ébullition est inférieure à 15 min et **en ce que** le résidu de distillation déterminé par pesée est converti en volume.

5. Dispositif pour la mise en oeuvre du procédé selon l'une des revendications 1 à 4 **caractérisé en ce qu'**une pompe est relié par une conduite à un récipient d'échantillon (7) pour l'introduction d'une quantité d'echantillon, **en ce que** le récipient d'échantillon (7) est disposé sur une base (8) déplaçable qui est reliée à un organe de positionnement (9) sous la forme d'un organe de levage par lequel le bord du récipient d'échantillon (7) peut être appuyé contre le bord de l'ouverture de raccordement d'une colonne de distillation (10) qui se raccorde au récipient d'échantillon (7) et **en ce que** la pompe (1) et l'espace de réception pour le condensat en particulier la condenseur (15), consistent en un matériau bon conducteur thermique, en particulier en métal, et sont reliés à un dispositif de régulation de la température.

6. Dispositif selon la revendication 5 **caractérisé en ce que** le dispositif de régulation de la température est agencé sous forme d'un dispositif de refroidissement et/ou de chauffage électrique, en particulier au moyen d'éléments Peltier (5,17), et est disposé, comme la pompe (1) et le dispositif de distillation, dans un boîtier portable commun.

7. Dispositif selon l'une des revendications 5 ou 6 **caractérisé en ce que** la pompe (1) et le condenseur (15) comportent des capteurs de température (6,16).

8. Dispositif selon l'une des revendications 5,6 ou 7 **caractérisé en ce que** l'organe de positionnement (9) pour le récipient d'échantillon (7) est agencé sous forme de motoréducteur.

9. Dispositif selon l'une des revendications 5 à 8 **caractérisé en ce que** le bord du récipient d'échantillon (7) et le bord de l'ouverture de raccordement de la colonne de distillation (10) sont agencés de manière conique, conique creuse ou bombée pour la liasion étanche aux gaz du récipient d'échantillon (7) avec le dispositif de distillation au moyen de la pression d'appui produite par l'organe de positionnement (9).

10. Dispositif selon l'une des revendications 5 à 9 **caractérisé en ce que** la colonne de distillation (10) est entourée par une isolation (12).

11. Dispositif selon l'une des revendications 5 à 10 **caractérisé en ce que** le condenseur (15) comporte un domaine axial de diamètre réduit en un matériau transparent pour la lumière, en particulier la lumière infrarouge, en particulier en verre, auquel se raccorde un domaine de plus grande largeur intérieure recevant un piston (22) mobile axialement.

12. Dispositif selon la revendication 11 **caractérisé en ce que** le piston (22) mobile axialement est relié à un organe de positionnement, en particulier un moteur pas-à-pas ou un motoréducteur, avec un actuateur rotatif (23), qui peut être entraîné selon les signaux d'un détecteur optique (21).

13. Dispositif selon l'une des revendications 5 à 12 **caractérisé en ce qu'**un générateur de signaux optiques (20) est disposé dans le domaine du domaine axial en matériaus transparent à la lumière et est agencé pour la détection du ménisque du liquide condensé et **en ce que** l'organe de positionnement du piston (22) peut être entraîné pour la correction de la position du ménisque.

14. Dispositif selon l'une des revendications 5 à 13 **caractérisé en ce que** le dispositif de distillation consiste en inox, en laiton ou en titane et le récipient d'échantillon (7) consiste en métal, de préférence en aluminium ou en cuivre.

15. Dispositif selon l'une des revendications 5 à 14 **caractérisé en ce qu'**un capteur de pression, en particulier un capteur de pression piézorésistif est prévu pour la détermination de la pression de l'air et **en ce que** le dispositif de distillation est agencé dans le domaine du condenseur (15) en liaison ouverte avec l'atmosphère.

16. Dispositif selon l'une des revendications 5 à 15 **caractérisé en ce qu'**une balance qui est agencée pour la pesée du résidu de distillation de la quantité d'échantillon est disposée dans le boîtier.

17. Dispositif selon l'une des revendications 5 à 16 **caractérisé en ce que** les valeurs mesurées sont transmises par des conduites à un microprocesseur qui est agencé pour l'évaluation et le calcul de résultats des mesures et **en ce qu'**il est prévu un indicateur ou un dispositif d'affichage pour les résultats des mesures.
